# EUROPEAN PATENT SPECIFICATION

(11) **EP 4 531 798 B1**
(45) Date of publication and mention of the grant of the patent: **24.09.2025**
(21) Application number: 23726489.0
(22) Date of filing: 12.05.2023
(51) Int. Cl.: A61K 8/11, B01J 13/02, C11D 3/50, A61K 8/64, A61K 8/73, C11D 3/37, C11D 3/22

(54) **METHOD OF PRODUCING BIODEGRADABLE MICROCAPSULES**
PROCÉDÉ DE PRODUCTION DE MICROCAPSULES BIODÉGRADABLES
VERFAHREN ZUR HERSTELLUNG VON BIOLOGISCH ABBAUBAREN MIKROKAPSELN

(30) Priority: 31.05.2022 EP 22176494
(43) Date of publication of application: 09.04.2025
(73) Proprietor: Unilever IP Holdings B.V., 6708 WH Wageningen (NL); Unilever Global IP Limited, Wirral, Merseyside CH62 4ZD (GB)
(72) Inventor: BOARDMAN, Christopher, 6708 WH Wageningen (NL); HENDERSON, Andrew, 6708 WH Wageningen (NL); JONES, Craig Warren, 6708 WH Wageningen (NL)
(74) Representative: McHugh, Paul Edward
(86) International application number: PCT/EP2023/062761
(87) International publication number: WO 2023/232432

(56) References cited:
- WO-A1-2020/188108
- WO-A1-2022/029490
- WO-A1-2023/274788
- CN-A- 101 240 224
- CN-A- 103 396 886
- CN-A- 113 444 566
- US-A1- 2009 258 812

## Description

### Field of the Invention

The present invention relates to microcapsules, and to a method of producing said microcapsules.

### Background of the Invention

Microcapsules are used in various applications to deliver active materials to a substrate. Microcapsules are known to provide various benefits, such as delayed release of the active material or protection of the active material from other ingredients in various compositions. Conventional microcapsules typically have a microcapsule wall formed of a synthetic polymer such as a melamine formaldehyde polymer or polyacrylate polymer.

WO 2020/131879 A1 discloses pea protein microcapsules with polyisocyanate in the shell which are covalently crosslinked. WO 2022/029490 A1 discloses a process for the preparation of a biodegradable protein- and/or polysaccharide- based microcapsule comprising the steps of: providing an internal non-aqueous phase comprising at least one first crosslinking agent and at least one hydrophobic active ingredient and optionally at least one further crosslinking agent; providing an external aqueous phase comprising at least one protein, such as pea protein and/or at least one polysaccharide, such as alginates; emulsifying or dispersing the internal non-aqueous phase in the external aqueous phase to obtain an oil-in-water emulsion or dispersion; first crosslinking by addition of at least one catalyst to obtain a microcapsule slurry; curing the microcapsule slurry at a temperature of at least 60 °C; and cooling.

There remains a need for improved microcapsules, specifically for improved feel of surfaces treated with microcapsules.

### Summary of the Invention

It has been found that a surface treated with the microcapsules of the invention has an improved feel to the consumer.

Disclosed, but not according to the invention, is a microcapsule, wherein the microcapsules have a core and a shell; the core comprising a core material selected from perfumes; the shell comprises plant protein and polysaccharide, wherein the polysaccharide is selected from: pectins, natural or chemically modified celluloses, xanthan gum, konjac gum, xyloglucan, guar gum, tara gum, locust bean gum, inulin, curdlan gum, diutan gum, alginate, gellan and carrageenan.

Preferably the microcapsules are biodegradable.

Preferably the microcapsules encapsulate biodegradable perfumes.

Preferably the protein used in the shell of the microcapsule is selected from pea protein.

Preferably the polysaccharide used in the shell of the microcapsule is selected from alginate, gellan, carrageenan, more preferably the polysaccharide is alginate.

Preferably the microcapsules comprise 1 to 80 wt.%, preferably from 2 to 70 wt.% of shell material.

Preferably the microcapsules further comprise a deposition aid attached to the shell of said microcapsule.

The invetion provides a method of producing biodegradable microcapsules, wherein the microcapsules have a core and a shell; the core comprising a core material selected from perfumes; the shell comprises pea protein and alginate; wherein the method does not involve chemically covalent crosslinking.

Preferably the method is selected from ionic crosslinking, charged polymer crosslinking or hydrogen bonding, more preferably ionic crosslinking.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive.

It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By microcapsule composition it is herein understood to mean the composition comprising microcapsules which is added to a further composition. The microcapsule composition may comprise only microcapsules or may be in the form of a slurry comprising microcapsules.

By microcapsule it is herein understood to mean the microcapsule (wall and core) i.e., without a solvent or slurry.

The microcapsules may be provided simply as microcapsules but preferably are provided in a microcapsule composition comprising microcapsules in a slurry. The microcapsules comprise a microcapsule core comprising a core material selected from perfumes and optionally benefit agents, and microcapsule wall encapsulating the core. The microcapsule wall comprises a wall polymer. Typically, the microcapsules comprise 10 wt.% to 98 wt.% core materials, 1 wt.% to 40 wt.% shell material comprising protein and polysaccharide.

The microcapsules are prepared by the method of claim 1.

The terms 'perfume' and 'fragrance' as used herein are used interchangeably to refer to the same material.

### Biodegradability

Wherein materials used herein are referred to as biodegradable, it is meant that they fulfill the requirements of biodegradability as defined in the OECD. Specifically, the biodegradability of materials is tested according to the OECD Standard 301F.

### Microcapsule

A microcapsule can be considered to be a capsule that encapsulates an active material. The microcapsules used herein have core material selected from perfumes and optionally benefit agents, and a shell comprising pea protein and alginate.

### Microcapsule shell materials

The microcapsule shell comprises plant protein and polysaccharide polymer according to claim 1. Protein and polysaccharide may be treated by various processes to provide derivatives, including but not limited to hydrolysis, condensation, functionalising such as ethoxylating, crosslinking, etc. The microcapsule wall materials are preferably in an aqueous solution. The microcapsule shell preferably comprises 20 wt.% to 100 wt.% protein and polysaccharide, more preferably 30 wt.% to 98 wt.%, more preferably 35 wt.% to 95 wt.%, and most preferably 65 wt.% to 90 wt.% by weight of the microcapsule shell.

As is conventional in the art, a "polypeptide" or "protein" is a linear organic polymer composed of amino acid residues bonded together in a chain, forming part of (or the whole of) a protein molecule. "Polypeptide" or "protein" as used herein means a natural polypeptide, polypeptide derivative, and/or modified polypeptide. The polypeptide may exhibit an average molecular weight of from 1,000 Da to 40,000,000 Da, preferably greater than 10,000 Da, more preferably, 100,000 Da, most preferably greater than 1,000,000 Da and preferably less than 3,000,000 Da.

Proteins include plant proteins.

Plant proteins include proteins selected from chickpea, pea proteins, potato proteins, brown rice proteins, white rice proteins, wheat proteins, barley proteins, pumpkin seed proteins, oat proteins, almond proteins, and combinations thereof. This includes derivatives of the aforementioned proteins.

. Plant proteins are proteins that accumulate in various plant tissues. Preferred plant proteins can be classified into two classes: seed or grain proteins and vegetable proteins. Seed/grain proteins are a set of proteins that accumulate to high levels in seeds/grains during the late stages of seed/grain development, whereas vegetable proteins are proteins that accumulate in vegetative tissues such as leaves, stems and, depending on plant species, tubers.

Examples of seed/grain/legumes storage proteins are proteins from: soya, lupine, pea, chickpea, alfalfa, horse bean, lentil, and haricot bean; from oilseed plants such as colza, cottonseed and sunflower; from cereals like wheat, maize, barley, malt, oats, rye and rice (e.g., brown rice protein), or a combination thereof.

Examples of vegetable protein are proteins form: potato or sweet potato tubers. The term plant protein is intended to include a plant protein isolate, plant protein concentrate, or a combination thereof. Plant protein isolates and concentrates are generally understood to be composed of several proteins. For example, pea protein isolates and concentrates may include legumin, vicilin and convicilin proteins. Similarly, brown rice protein isolates may include albumin, globulin and glutelin proteins. The term "plant protein" is also intended to include a partially or completely modified or denatured plant storage protein. Individual polypeptides (e.g., legumin, vicilin, convicilin, albumin, globulin or glutelin) may also be used in preparation of microcapsules of this invention.

A native protein maybe preferred. However, the process may include a step of denaturing the protein by pH adjustment, heat, or adding a chaotropic agent to the oil-in-water emulsion or to the protein before adding to the oil-in-water emulsion.

Denaturation is a process in which proteins (polypeptides) lose the quaternary structure, tertiary structure, and secondary structure present in their native state, by application of a denaturation condition. During denaturation, proteins change their conformational structure by unfolding, thereby making amine and hydroxyl groups available for crosslinking to form a microcapsule wall. Exemplary conditions for protein denaturation include, but are not limited to, radiation, exposure to heat or cold, changes in pH with an acid or base, exposure to denaturing agents such as detergents, inorganic salt, organic solvent (e.g., alcohol, ethyl acetate, and chloroform), urea, or other chaotropic agents, or mechanical stress including shear. Exemplary chaotropic agents are guanidine salts (e.g., guanidine hydrochloride and guanidine carbonate), urea, polysorbate, sodium benzoate, vanillin, o-cresol, phenol, propanol, formamide, ethanol, fructose, ammonium sulfate, ammonium chloride, ammonium nitrate, ammonium phosphate, potassium sulfate, potassium chloride, potassium iodide, potassium nitrate, potassium phosphate, sodium sulfate, sodium chloride, sodium bromide, sodium nitrate, sodium phosphate, guanidine thiocyanate, xylose, glycerol, benzyl alcohol, ethyl acetate, triton X-100, ethyl acetate, cetyltrimethylammonium halide, acetone, sodium dodecyl sulfate (SDS), hydrochloric acid, sulfuric acid, polyethylene glycol, glutaraldehyde, and combinations thereof.

Any amount of the chaotropic agent can be used. It may be preferred that the protein is denatured with a chaotropic agent so that 20 wt. % to 100 wt.% preferably 40 wt. % to 100 wt. %, more preferably 60 wt.% to 100 wt.%, most preferably 90 wt.% to 100 wt.% of the protein used in the preparation of the microcapsules is denatured.

The protein used in the microcapsule can also be derivatized or modified (e.g., derivatized or chemically modified). For example, the protein can be modified by covalently attaching sugars, lipids, cofactors, peptides, or other chemical groups including phosphate, acetate, methyl, and other natural or unnatural molecule.

The plant protein used in the method of claim 1 is pea protein.

Polysaccharides are a class of carbohydrates comprising multiple monosaccharide units.

"Polysaccharide" as used herein means a natural polysaccharide, polysaccharide derivative, and/or modified polysacharide.

Examples of polysaccharides are pectins (e.g. various sources such as citrus, beets or apples), celluloses (natural or chemically modified, such as hydroxyethyl cellulose), xanthan gum, konjac gum, xyloglucan, guar gum, tara gum, locust bean gum, inulin, curdlan gum, diutan gum, alginate, gellan and carrageenan.

Particular polysaccharides include: alginate, gellan and carrageenan.

The polysaccharide used in the microcapsule can also be derivatized or modified (e.g., derivatized or chemically modified). For example, the protein can be modified by covalently attaching sugars, lipids, cofactors, peptides, or other chemical groups including phosphate, acetate, methyl, and other natural or unnatural molecule. Examples of polysaccharide derivatives include: starch glycolate, carboxymethyl starch, hydroxyalkyl cellulose and crosslinked modified cellulose.

The microcapsule preferably comprises from 0.1 wt.% to 90 wt.% microcapsule shell, preferably 1 wt.% to 80 wt.%, more preferably 2 wt.% to 70 wt.% microcapsule shell by weight of the microcapsule. Preferably this level of shell material is calculated just by the level of protein and polysaccharide, by weight of the microcapsule.

The microcapsule wall polymers described herein are preferably crosslinked, however the method to produce the microcapsules does not involve chemically covalent crosslinking. Preferably the method is selected from ionic crosslinking, charged polymer crosslinking or hydrogen bonding, more preferably ionic crosslinking.

Suitable methods of crosslinking include: ionic crosslinking, charged polymer crosslinking or hydrogen bonding, and internal crosslinking within the microcapsule wall polymer structures (including the formation of a coacervate). Preferably the method involves ionic crosslinking, e.g. salt bridge crosslinking. Where a cross linking agent is used, such as ionic crosslinking agents the crosslinking agent is preferably present at a level of 0.1 wt.% to 10 wt.% by weight of the microcapsule.

A crosslinking agent suitable for use in the present invention are ionic crosslinking agents. Ionic crosslinking agents are multivalent ions which are capable of forming salt bridges with the functional groups of the protein or polysaccharide polymers. Suitable ionic crosslinking agents include calcium, copper, aluminum, magnesium, strontium, barium, zinc, tin, organic cations, poly(amino acids), poly(ethyleneimine), poly(vinylamine), poly(allyl amine) and polysaccharides, and multivalent anions are selected from the group consisting of dicarboxylic acids, sulfate ions, carbonate ions, poly(acrylic acids), poly(methacrylic acids), copolymers of acrylic acid or methacrylic acid, sulfonated poly(styrene) and poly(styrene) with carboxylic acid groups and mixtures thereof. Particularly preferred are calcium salts, magnesium, sodium, potassium, strontium, barium, zinc.

Internal cross linking is cross linking between the microcapsule wall polymers, without the use of a crosslinking agent. The internal crosslinking maybe crosslinking with the same polymer (i.e. a polymer with both positive and negative charges) or between two different polymers forming the microcapsule wall. When two different polymers of opposite charges are utilised, this is referred to as a coacervate formed by coacervation.

Preferably a coacervate is formed between a first protein or polysaccharide of one charge and a second protein or polysaccharide of an opposite charge. The ratio between polymer with a positive charge and polymer with a negative charge is preferably between 10/0.1 to 0.1/10, more preferably between 10/1 and 1/10 and most preferably between 6/1 and 1/6.

An example of a protein with a positive charge maybe gelatin and the protein or polysaccharide with a negative charge maybe selected from the group consisting of gum arabic, xanthan, alginate salts, pectinate salts, carrageenan, polyacrylic and methacrylic acid, xanthan gum and plant gums and/or mixtures thereof.

Microcapsules which are internally cross linked, i.e. have electrostatic interaction between positive and negative monomers within the microcapsule wall polymers, can be further crosslinked or 'hardened' using salt bridges as described above.

The microcapsule may optionally comprise further crosslinking agents. The further crosslinking agent maybe selected from the group consisting of transglutaminase, peroxidase, secondary plant substances selected from the group consisting of polyphenols, in particular tannin, gallic acid, ferulic acid, hesperidin, cinnamaldehyde, vanillin, carvacrol, and mixtures of two or more of the aforementioned crosslinking agents.

### Microcapsule core materials

The core material is selected from perfumes.

The core may also be referred to as the internal phase. The core of the microcapsule comprises active material and optionally further comprises solvents and/or crosslinking agents as described above. The core is preferably non-aqueous.

The microcapsules encapsulate perfume in the core (i.e. the core material is perfume in whole or in part). Preferably the perfume is biodegradable, wherein the biodegradability of the perfume is tested according to the OECD Standard 301F.

The internal non-aqueous phase may preferably comprise from 20 to 80 wt.%, preferably from 25 to 75 wt.% and even more preferably from 33 to 50 wt.% active material to be encapsulated and preferably from 0.1 to 5 wt.%, preferably from 0.15 to 3.5 wt.% and even more preferably from 0.5 to 2.5 wt.% crosslinking agent and the remaining composition solvent.

The benefit agents may be dissolved in a solvent. Examples of suitable solvents include vegetable oils, glycerides, esters of fatty acids and branched alcohols, hydrocarbon, etc. specific examples include: diethyl phthalate, isopropyl myristate, Abalyn^{®} (rosin resins, available from Eastman), benzyl benzoate, ethyl citrate, limonene or other terpenes, triacetin or isoparaffins, preferably Abalyn^{®}, benzyl benzoate, limonene or other terpenes, isoparaffins, or combinations thereof. Preferably, if present, the solvent is 0 to 30 wt.% of the active material, more preferably 0 to 20 wt.% and most preferably 0 to 10 wt.% of the active material.

The microcapsule core material comprises perfume. Perfume components are well known in the art. Useful perfume components may include materials of both natural and synthetic origin. They include single compounds and mixtures. Specific examples of such components may be found in the current literature, e.g., in Fenaroli's Handbook of Flavor Ingredients, 1975, CRC Press; Synthetic Food Adjuncts, 1947 by M. B. Jacobs, edited by Van Nostrand; or Perfume and Flavor Chemicals by S. Arctander 1969, Montclair, N.J. (USA). These substances are well known to the person skilled in the art of perfuming, flavouring, and/or aromatizing consumer products.

Particularly preferred perfume components are blooming perfume components and substantive perfume components. Blooming perfume components are defined by a boiling point less than 250°C and a LogP greater than 2.5. Preferably encapsulated perfume compositions comprise at least 20 wt.% blooming perfume ingredients, more preferably at least 30 wt.% and most preferably at least 40 wt.% blooming perfume ingredients. Substantive perfume components are defined by a boiling point greater than 250°C and a LogP greater than 2.5. Preferably encapsulated perfume compositions comprises at least 10 wt.% substantive perfume ingredients, more preferably at least 20 wt.% and most preferably at least 30 wt.% substantive perfume ingredients. Boiling point is measured at standard pressure (760 mm Hg). Preferably a perfume composition will comprise a mixture of blooming and substantive perfume components. The perfume composition may comprise other perfume components.

It is commonplace for a plurality of perfume components to be present in a microcapsule. In the compositions for use in the present invention it is envisaged that there will be three or more, preferably four or more, more preferably five or more, most preferably six or more different perfume components in a microcapsule. An upper limit of 300 perfume components may be applied.

Preferably the amount of encapsulated active material is from 5 wt.% to 95 wt.%, preferably 10 wt.% to 90 wt.% more preferably 15 wt.% to 85 wt.%, and most 20 wt.% to 80 wt.% by weight of the microcapsule.

### Deposition aids

Preferably the microcapsule further comprises one or more deposition aids attached to the shell of the microcapsule.

If used, then preferably the deposition aid is from the group consisting of trimonium, methacrylamidopropyl trimethyl ammonium, acrylamidopropyl trimethylammonium, acrylamide, acrylic acid, dimethyl ammonium, xlylose, galactose, hydroxypropylated glucose, chitosan, hydroxyethylated glucose, hydroxymethylated glucose, vinylamine, ethylenimine, functionalized branched polyethylenimine, vinylformamide, vinylpyrollidone, caprolactone, catechol, vinylalcohol, chitosan, polyquatemium-4, polyquatemium-5, polyquatemium-6, polyquatemium-7, polyquatemium-10, polyquatemium-11, polyquatemium-16, polyquatemium-22, polyquatemium-24, polyquatemium-28, polyquatemium-37, polyquatemium-39, polyquatemium-44, polyquatemium-46, polyquatemium-47, polyquatemium-53, polyquatemium-55, polyquatemium-67, polyquatemium-68, polyquatemium-69, polyquatemium-73, polyquatemium-74, polyquatemium-77, polyquatemium-78, polyquatemium-79, polyquatemium-80, polyquatemium-81, polyquatemium-82, polyquatemium-86, polyquatemium-88, polyquatemium-101, polyquatemium-79 hydrolyzed keratin, polyvinylamine, polyethyleneimine, a copolymer of vinylamine and vinylformamide, a copolymer of acrylamide and 3-methacryloylaminopropyl trimethylammonium, a 3-acrylamidopropyl trimethylammonium polymer or its copolymer, a diallyldimethylammonium-chloride polymer and its copolymer, a polysaccharide with saccharide unit functionalized with hydroxypropyl trimmonium, ethyltrimonium chloride methacrylate and hydrolyzed wheat protein copolymer, alkyl-monium hydroxypropyl hydrolyzed protein, and combinations thereof.

More preferably, any deposition aid used is biodegradable according to the OECD Standard 301F.

### Microcapsule composition

As used herein, the term "microcapsule" refers to capsules preferably having a median particle size d(50) from 0.1 to 1000 micrometers, preferably from 0.1 to 300 micrometers, more preferably from 1 to 200 micrometers, e.g. from 20 to 200 micrometers.

The median particle size d(50) is determined by laser diffraction analysis, for example by using a Horiba LA 940 or Mastersizer 3000 from Malvern using the "Mie Scattering Theory" evaluation. Median values are defined as the value where half of the population resides above this point, and half resides below this point. For particle size distributions the median is called the "d(50)" or "D50".

The microcapsules can be positively or negatively charged with a zeta potential of preferably - 200 mV to +200 mV, more preferably 25 mV to 200 mV, and most preferably 40 mV to 100 mV. Preferably, the microcapsules are positively charged. Zeta potential is a measurement of electrokinetic potential in the microcapsule. From a theoretical viewpoint, zeta potential is the potential difference between the water phase (i.e., the dispersion medium) and the stationary layer of water attached to the surface of the microcapsule. The zeta potential can be calculated using theoretical models and an experimentally-determined electrophoretic mobility or dynamic electrophoretic mobility. The zeta potential is conventionally measured by methods such as microelectrophoresis, or electrophoretic light scattering, or electroacoustic phenomena. For more detailed discussion on measurement of zeta potential, see Dukhin and Goetz, "Ultrasound for characterizing colloids", Elsevier, 2002.

The microcapsule composition of this invention can be a slurry containing a solvent, preferably water. The microcapsules are preferably present in the slurry as 0.1 wt.% to 80 wt.%, more preferably 1 wt.% to 65 wt.% and most preferably 5 wt.% to 45 wt.% by weight of the microcapsule composition. The slurry may comprise a thickening or suspending agent such as xanthan gum, carboxymethyl cellulose (CMC), microcrystalline cellulose (MCC) or guar gum. Alternatively the microcapsule composition of this invention can also be dried, e.g., spray dried, heat dried, and belt dried, to a solid form.

The microcapsule composition maybe purified by washing the capsule slurry with water until a neutral pH (pH of 6 to 8) is achieved. For the purposes of the present invention, the capsule suspension can be washed using any conventional method including the use of a separatory funnel, filter paper, centrifugation and the like. The capsule suspension can be washed one, two, three, four, five, six, or more times until a neutral pH, e.g., pH 6-8 and 6.5-7.5, is achieved. The pH of the purified capsules can be determined using any conventional method including, but not limited to pH paper, pH indicators, or a pH meter. In certain embodiments of this invention, the purification of the capsules includes the additional step of adding a salt to the capsule suspension prior to the step of washing the capsule suspension with water. Exemplary salts of use in this step of the invention include, but are not limited to, sodium chloride, potassium chloride or bi-sulphite salts.

### Preparation of the microcapsules

When preparing the microcapsule, the polymers may be provided in a solvent. Suitable solvents for preparing the microcapsule wall include water or mixtures of water with at least one water-miscible organic solvent. Suitable organic solvents include glycerol, 1 ,2-propanediol, 1 ,3-propanediol, ethanediol, diethylene glycol, triethylene glycol, and other analogues. Preferably the solvent is water.

A stabiliser may also be present. A stabiliser maybe be selected from acrylic co-polymers, preferably with sulphonate groups, copolymers of acrylamides and acrylic acid, copolymers of alkyl acrylates and N-vinylpyrrolidone, such as LUVISKOL^{®} K15, K30 or K90 (BASF); sodium polycarboxylates, sodium polystyrene sulfonates, vinyl and methyl vinyl ether-maleic acid anhydride copolymers as well as ethylene, isobutylene or styrene-maleic acid anhydride copolymers, microcrystalline cellulose, which is commercially available, for example, under the name VIVAPUR^{®}, diutan gum, xanthan gum or carboxymethyl celluloses.

The crosslinking of the microcapsule preferably involves a catalyst. The catalyst may be present in the microcapsule core active material or added separately to the oil-in-water emulsion or dispersion. A preferred catalyst for the formation of microcapsules according to the present invention is diazabicyclo[2.2.2]octane (DABCO), also known as triethylenediamine (TEDA).

Also suitable are catalysts based on bismuth or tin, transglutaminase, peroxidase, secondary plant compounds selected from the group, which consists of polyphenols, in particular tannin, gallic acid, ferulic acid, hesperidin, cinnamaldehyde, vanillin, carvacrol, and mixtures thereof. Alternatively, or additionally the formation of the microcapsules may be catalysed by heating the oil-in-water emulsion or dispersion. For example, heating to a temperature range of a temperature in the range of 60 °C to 90 °C.

Various suitable methods of production may be implemented to produce the microcapsules suitable for use in the present invention, provided they do not involve chemically covalent crosslinking.

One suitable method is interfacial polymerization. An example of interfacial polymerization involves the steps of:
(i) Providing the microcapsule core materials including at least one first crosslinking agent, wherein the crosslinking agent is substantially dissolved together with the core materials. this is the oil phase. A non-aqueous solvent may optionally be present;
(ii) Providing the microcapsule wall materials comprising at least one protein and at least one polysaccharide. This is the aqueous phase. An aqueous solvent may optionally be present;
(iii) Emulsifying or dispersing the microcapsule core materials with the microcapsule wall materials. Preferably in a ratio of from 70 : 30 to 60 : 40, preferably in a range from 30 : 70 to 60 : 40. Optionally an emulsifier may be used. Emulsification may be achieved by use of highspeed mixing. After completion of this stage, an oil-in-water emulsion or dispersion is present in which the internal phase with the active materials to be encapsulated is finely emulsified or dispersed in the external wall material phase in the form of droplets;
(iv) First crosslinking optionally by the addition of at least one catalyst to obtain a microcapsule slurry, optionally with the addition of further protein and/or polysaccharide polymers;
(v) Curing the microcapsule slurry, preferably at a temperature of at least 60°C, preferably for at least 30 minutes, followed by cooling;
(vi) Optionally drying, using methods such as spray drying, filtration, or freeze drying.

An alternative method involves 3D printing the microcapsules. Both the microcapsule shell and microcapsule core can be printed using a printing system. See WO2016172699A1. The printing steps generally include depositing the active materials and the microcapsule shell material in a layer-by-layer fashion, preferably through separate printer heads.

### In use

Fabric care may be defined by various parameters, in particular fabric hand, softness and resilience. These are measures which decrease as a fabric is damaged. In other words, fabric care may be considered the prevention of damage or the maintenance of fabric. Fabric hand is defined as the estimated quality of a fabric, evaluated when a consumer touches a fabric. Important components of fabric hand include smoothness, compressibility and elasticity of the textile sample. The compositions described herein may also provide improved softening and/or resilience to fabrics treated with the composition. Resilience is the ability of a fiber to spring back to its natural position after folding, creasing or deformation. An improvement in fabric hand, resilience or softening may be measured using a sensorial evaluation. Alternatively, machine measurements can be used. Suitable machines for measuring the relative hand value, resilience or softness are a PhabrOmeter^{®} supplied by Nu Cybertek, Inc. An improvement in fabric hand can be measured using AATCC TM202-2012 (2020), Relative Hand Value of Textiles: Instrumental Method.

### Examples

### Example 1

### Preparation of microcapsules

The following microcapsules were used to assess the effects of the compositions described herein:
- Microcapsules A (reference example)
   - Melamine formaldehyde microcapsules
- Microcapsules B (reference example)
   - Pea protein and crosslinked with polyisocyanate (Poly[(phenyl isocyanate-co-formaldehyde])
- Microcapsules 1 - Pea protein and sodium alginate mix and with salt crosslinking

The same model fragrance composition was used in all microcapsules.

Microcapsule A was prepared by dispersing the formalin in water and adjusting the pH to 9. Melamine and 0.28g of sodium chloride were added. The mixture was heated to ~65°C for an hour. Further water was added and the solution heated to ~75°C. The pH was adjusted to 4. The fragrance was then added with homoginisation. Heating and stirring was continued for 3 hours. The mixture was then cooled and the pH adjusted to 7.

**Table 1: Microcapsule A composition**

| **Component** | **Amount** | **% by weight of microcapsule** |
|---|---|---|
| *Core:* | | (75%) |
| *Fragrance* | 22.6 g | 75% |
| *Shell:* | | (25%) |
| *Melamine* | 4.3 g | 14.2% |
| *Formalin (formaldehyde)* | 3.2 g | 10.6% |
| Total | 30.1 g | (100%) |

Microcapsule B was prepared as follows: an oil phase was first prepared by mixing 23.2g of a model fragrance and 4.8g of caprylic/capric triglyceride, 0.38g of benzyl benzoate and an aromatic polyisocyanate 0.38g. In a separate beaker, an aqueous solution was obtained by mixing 1.182g of a pea protein isolate in 170g of water. The oil phase was then emulsified into the aqueous phase to form an oil-in-water emulsion using an ultra turrax mixer at a shear rate of 5000 Revolutions Per Minute (RPM). The oil-in-water emulsion was then cured at 55°C for 2 hours.

**Table 2: Microcapsule B composition**

| **Component** | **Amount** | **% by weight of microcapsule** |
|---|---|---|
| *Core:* | | (94.81%) |
| *Fragrance* | 23.226 g | 77.43% |
| *Caprylic*/*capric triglyceride* | 4.839 | 16.13 |
| *Benzyl Benzoate* | 0.375 | 1.25 |
| *Shell:* | | (5.19%) |
| *Pea Protein isolate* | 1.182 g | 3.94% |
| *Polyisocyanate* | 0.375 g | 1.25% |
| Total | 30 g | (100%) |

Microcapsule B are covalently crosslinked polyisocyanate microcapsules as described in WO 2020/131879 A1.

Microcapsule 1 (according to the invention) was prepared as follows: a solution of pea protein was prepared in demineralised water and the pH was adjusted to pH9 using a few drops of sodium carbonate solution. This was heated to 85 °C and mixed at 300RPM mixed on a tornado mixer for 2hrs. After 2hrs the pH was dropped to pH7 with a few drops of 2M HCl solution and 1.5g of sodium alginate was added.

This was further mixed at 300RPM for 90min until homogeneous and then was left overnight to cool down. Once cool the next day 22.5g of fragrance oil was added and mixed at 300RPM for 1hr. The prepolymer mix was then added into an 80ml solution containing tween 80, calcium chloride and DI water at varying concentrations. The solution was homogenised to produce a suspension.

**Table 2: Microcapsule 1 composition**

| **Component** | **Amount** | **% by weight of microcapsule** |
|---|---|---|
| *Core:* | | (72.4%) |
| *Fragrance* | 22.5 g | 72.4% |
| *Shell:* | | (27.6%) |
| *Pea Protein isolate* | 6 g | 19.3% |
| *Sodium Alginate* | 1.5 g | 4.8% |
| *Calcium Ions* | 1.1g | 3.5% |
| Total | 31.1 g | (100%) |

### Preparation of fabric conditioner

**Table 4: Fabric conditioner composition**

| **Ingredient** | **wt.% active** | | |
|---|---|---|---|
| | **A** | **B** | **1** |
| Fabric softening active¹ | 6 | 6 | 6 |
| Perfume microcapsule A | 5 | - | - |
| Perfume microcapsule B | - | 5 | - |
| Perfume microcapsule 1 | - | - | 5 |
| Minors | <1 | <1 | <1 |
| Water | To 100 | To 100 | To 100 |

### Fabric softening active¹ - Dialkyloxyethyl Hydroxyethyl Methyl Ammonium Methyl sulphate

The fabric conditioner was prepared by melting the fabric softening active at ~65 °C. Water was heated to ~40°C. The minors and fabric softening active were added with stirring. The water was then cooled slightly. The composition was then cooled to room temperature. The perfume microcapsules were then added to the compositions. 2 wt.% is based of weight of microcapsules.

### Wash method

The washes took place in a Terg-O-Tometer pot. 0.5 ml of the test fabric conditioner composition and 1 L of water were added to the tergo pot, followed by 40g of knitted cotton. The test monitors were rinsed in the liquor for 10 minutes and spun for 30 seconds. The fabrics were then hung to dry in ambient conditions.

### Analysis

The properties of the fabrics were assessed using a Phabrometer by NUCybertek*.

A 11.3cm disc is cut out from the test fabric. The fabric measurements were performed using a Phabrometer and according to AATCC standard TM202.

10 repeat measurements were taken for each fabric, and the results averaged.

**Table 5: Results**

| **Microcapsule type** | **Relative hand value** |
|---|---|
| Microcapsule A | 1.4682 |
| Microcapsule B | 1.5633 |
| Microcapsule 1 | 1.9391 |

A higher score indicates an improved relative hand value. The microcapsules according to the present invention provide improved relative hand value compared to conventional melamine formaldehyde microcapsules as well as covalently crosslinked polyisocyanate microcapsules as described in WO 2020/131879 A1.

### Example 2

Microcapsules B and 1 were produced as for example 1.

### Preparation of fabric conditioner

**Table 6: Fabric conditioner composition**

| **Ingredient** | **wt.% active** | |
|---|---|---|
| | **B** | **1** |
| Fabric softening active¹ | 6 | 6 |
| Perfume microcapsule B | 5 | - |
| Perfume microcapsule 1 | - | 5 |
| Minors | <1 | <1 |
| Water | To 100 | To 100 |

### Fabric softening active¹ - Dialkyloxyethyl Hydroxyethyl Methyl Ammonium Methyl sulphate

The fabric conditioner was prepared by melting the fabric softening active at ~65 °C. Water was heated to ~40°C. The minors and fabric softening active were added with stirring. The water was then cooled slightly. The composition was then cooled to room temperature. The perfume microcapsules were then added to the compositions. Wt.% is based of weight of microcapsules.

### Wash method

The washes took place in a Terg-O-Tometer pot. 0.5 ml of the test fabric conditioner composition and 1 L of water were added to the tergo pot, followed by 40g of knitted cotton. The test monitors were rinsed in the liquor for 10 minutes and spun for 30 seconds. The fabrics were then hung to dry in ambient conditions.

### Analysis

The properties of the fabrics were assessed using a Phabrometer by NUCybertek*. A 11.3cm disc is cut out from the test fabric. The fabric measurements were performed using a Phabrometer and according to AATCC standard TM202.

10 repeat measurements were taken for each fabric, and the results averaged.

**Table 7: Results**

| **Microcapsule type** | **Drape** | **Resilience** |
|---|---|---|
| Microcapsule B | 20.85 | 60.58 |
| Microcapsule 1 | 18.68 | 56.96 |

A lower number indicates an improved fabric performance. The fabrics treated with a product comprising microcapsules having an ionic crosslinking agent have better fabric shape and drape.

### Example 3

Further microcapsules made according to the exemplified method below with any minor deviations from the general method noted:-

### General Method

A solution of pea protein was prepared in demineralised water and the pH was adjusted to pH 9 using a few drops of sodium carbonate solution. This was heated to 85°C and mixed at 300RPM mixed on a tornado mixer for 2hrs. After 2hrs the pH was dropped to pH 7 with a few drops of 2M HCl solution and 1g of polysaccharide was added.

This was further mixed at 300RPM for 90min until homogeneous and then was left overnight to cool down. Once cool the next day 15g of fragrance oil was added and mixed at 300RPM for 1hr. The prepolymer mix was then added into a 40ml solution containing tween 80, calcium chloride and DI water at varying concentrations. The solution was homogenised to produce a suspension.
Microcapsules 2 dispersed in 80 g in demineralised water containing 2% emulsifying agent (Tween 80) and 2% CaCl₂.2H₂O
Homogenisation rate 20000 RPM, heating temperature 85°C, 2hrs

**Table 8: Microcapsule 2 composition**

| **Component** | **Amount** | **% by weight of microcapsule** |
|---|---|---|
| *Core:* | | (72.5%) |
| *Fragrance* | 15 g | 72.5% |
| *Shell:* | | (27.5%) |
| *Pea Protein isolate* | 4 g | 19.3% |
| *Sodium Alginate* | 1 g | 4.8% |
| *Calcium Ions* | 0.7g | 3.4% |
| Total | 20.7 g | (100%) |

Microcapsules 3 dispersed in 80 g in demineralised water containing 2% emulsifying agent (Tween 80) and 2% CaCl₂.2H₂O
Homogenisation rate 20000 RPM, heating temperature 85C, 2hrs

**Table 9: Microcapsule 3 composition**

| **Component** | **Amount** | **% by weight of microcapsule** |
|---|---|---|
| *Core:* | | (72.7%) |
| *Fragrance* | 15 g | 72.0% |
| *Lactam* | 0.14 g | 0.7% |
| *Shell:* | | (27.3%) |
| *Pea Protein isolate* | 4 g | 19.2% |
| *Sodium Alginate* | 1 g | 4.8% |
| *Calcium Ions* | 0.7g | 3.4% |
| Total | 20.8g | (100%) |

Microcapsules 4 dispersed in 80 g in demineralised water containing 2% emulsifying agent (Tween 80) and 2% CaCl₂.2H₂O
Homogenisation rate 20000 RPM, heating temperature 85°C, 2hrs

**Table 10: Microcapsule 4 composition**

| **Component** | **Amount** | **% by weight of microcapsule** |
|---|---|---|
| *Core:* | | (72.7%) |
| *Ethyl Lactate* | 15 g | 72.5% |
| *Lactam* | 0.04 g | 0.2% |
| *Shell:* | | (27.3%) |
| *Pea Protein isolate* | 4 g | 19.3% |
| *Sodium Alginate* | 1 g | 4.8% |
| *Calcium Ions* | 0.7g | 3.4% |
| Total | 20.7 g | (100%) |

Microcapsules 5 dispersed in 80 g in demineralised water containing 2% emulsifying agent (Tween 80) and 2% CaCl₂.2H₂O
Homogenisation rate 20000 RPM, heating temperature 85°C, 2hrs

**Table 11; Microcapsule 5 composition**

| **Component** | **Amount** | **% by weight of microcapsule** |
|---|---|---|
| *Core:* | | (73.7%) |
| *Fragrance* | 30 g | 73.7% |
| *Shell:* | | (26.3%) |
| *Pea Protein isolate* | 8 g | 19.7% |
| *Sodium Alginate* | 2 g | 4.9% |
| *Calcium Ions* | 0.7g | 1.7% |
| Total | 40.7 g | (100%) |

Microcapsules 6 dispersed in 80 g in demineralised water containing 2% emulsifying agent (Tween 80) and 2% CaCl₂.2H₂O
Homogenisation rate 20000 RPM, heating temperature 85°C, 2hrs

**Table 12: Microcapsule 6 composition (reference example)**

| **Component** | **Amount** | **% by weight of microcapsule** |
|---|---|---|
| *Core:* | | (72.5%) |
| *Fragrance* | 15 g | 72.5% |
| *Shell:* | | (27.5%) |
| *Pea Protein isolate* | 4 g | 19.3% |
| *Iota Carrageenan* | 1 g | 4.8% |
| *Calcium Ions* | 0.7g | 3.4% |
| Total | 20.7 g | (100%) |

Microcapsule 7 dispersed in 300 g in demineralised water containing 1% emulsifying agent (Tween 80) and 2% CaCl₂.2H₂O
Homogenisation rate 20000 RPM, heating temperature 85°C, 2hrs

**Table 13: Microcapsule 7 composition**

| **Component** | **Amount** | **% by weight of microcapsule** |
|---|---|---|
| *Core:* | | (38.5%) |
| *Fragrance* | 2 g | 38.5% |
| *Shell:* | | (61.5%) |
| *Pea Protein isolate* | 2 g | 38.5% |
| *Sodium Alginate* | 0.5 g | 9.6% |
| *Calcium Ions* | 0.7g | 13.5% |
| Total | 5.2 g | (100%) |

Microcapsules 8 dispersed in 300 g in demineralised water containing 1.5% emulsifying agent (Tween 80) and 2% CaCl₂.2H₂O
Homogenisation rate 20000 RPM, heating temperature 85°C, 2hrs

**Table 14: Microcapsule 8 composition (reference example)**

| **Component** | **Amount** | **% by weight of microcapsule** |
|---|---|---|
| *Core:* | | (33.9%) |
| *Fragrance* | 2 g | 33.9% |
| *Shell:* | | (66.1%) |
| *Pea Protein isolate* | 2 g | 33.9% |
| *Iota Carageenan* | 0.5 g | 8.5% |
| *Calcium Ions* | 1.4g | 23.7% |
| Total | 5.9 g | (100%) |

Microcapsule 9 dispersed in 300 g in demineralised water containing 1.5% emulsifying agent (Tween 80) and 2% CaCl₂.2H₂O
Homogenisation rate 20000 RPM, heating temperature 85°C, 2hrs

**Table 15: Microcapsule 9 composition (reference example)**

| **Component** | **Amount** | **% by weight of microcapsule** |
|---|---|---|
| *Core:* | | (33.9%) |
| *Fragrance* | 2 g | 33.9% |
| *Shell:* | | (66.1%) |
| *Pea Protein isolate* | 2 g | 33.9% |
| *Kappa Carageenan* | 0.5 g | 8.5% |
| *Calcium Ions* | 1.4g | 23.7% |
| Total | 5.9 g | (100%) |

Microcapsules 10 dispersed in 300 g in demineralised water containing 1% emulsifying agent (Tween 80) and 2% CaCl₂.2H₂O
Homogenisation rate 20000 RPM, heating temperature 85C, 2hrs

**Table 16: Microcapsule 10 composition (reference example)**

| **Component** | **Amount** | **% by weight of microcapsule** |
|---|---|---|
| *Core:* | | (32.3%) |
| *Fragrance* | 2 g | 32.3% |
| *Shell:* | | (68.7%) |
| *Pea Protein isolate* | 2 g | 32.3% |
| *Gellan Gum* | 0.5 g | 8.1% |
| *Calcium Ions* | 1.7g | 28.2% |
| Total | 6.2 g | (100%) |

Microcapsule 11 dispersed in 150 g in demineralised water containing 2% emulsifying agent (Tween 80) and 2% CaCl₂.2H₂O
Homogenisation rate 20000 RPM, heating temperature 85C, 2hrs

**Table 17: Microcapsule 11 composition**

| **Component** | **Amount** | **% by weight of microcapsule** |
|---|---|---|
| *Core:* | | (73.8%) |
| *Fragrance* | 16.9 g | 73.8% |
| *Shell:* | | (26.2%) |
| *Pea Protein isolate* | 4.2 g | 18.3% |
| *Sodium Alginate* | 1.1 g | 4.8% |
| *Calcium Ions* | 0.7g | 3.1% |
| Total | 22.9 g | (100%) |

## Claims

1. A method of producing biodegradable microcapsules, wherein the microcapsules have a core and a shell; the core comprising a core material selected from perfumes; the shell comprises pea protein and alginate;
wherein the method does not involve chemically covalent crosslinking, preferably the method is selected from ionic crosslinking, charged polymer crosslinking or hydrogen bonding, more preferably ionic crosslinking.

2. A method of producing microcapsules according to claim 1, wherein the method is selected from ionic crosslinking, charged polymer crosslinking or hydrogen bonding, preferably ionic crosslinking.

3. A method of producing microcapsules according to claim 1 or claim 2, wherein the microcapsules encapsulate biodegradable perfumes.

4. A method of producing microcapsules according to any preceding claim, wherein the microcapsules comprise 1 to 80 wt.%, preferably from 2 to 70 wt.% of shell material.

5. A method of producing microcapsules according to any preceding claim, wherein the microcapsules further comprise a deposition aid attached to the shell of said microcapsule, preferably the deposition aid is selected from the group consisting of trimonium, methacrylamidopropyl trimethyl ammonium, acrylamidopropyl trimethylammonium, acrylamide, acrylic acid, dimethyl ammonium, xlylose, galactose, hydroxypropylated glucose, chitosan, hydroxyethylated glucose, hydroxymethylated glucose, vinylamine, ethylenimine, functionalized branched polyethylenimine, vinylformamide, vinylpyrollidone, caprolactone, catechol, vinylalcohol, chitosan, polyquatemium-4, polyquatemium-5, polyquatemium-6, polyquatemium-7, polyquatemium-10, polyquatemium-11, polyquatemium-16, polyquatemium-22, polyquatemium-24, polyquatemium-28, polyquatemium-37, polyquatemium-39, polyquatemium-44, polyquatemium-46, polyquatemium-47, polyquatemium-53, polyquatemium-55, polyquatemium-67, polyquatemium-68, polyquatemium-69, polyquatemium-73, polyquatemium-74, polyquatemium-77, polyquatemium-78, polyquatemium-79, polyquatemium-80, polyquatemium-81, polyquatemium-82, polyquatemium-86, polyquatemium-88, polyquatemium-101, polyquatemium-79 hydrolyzed keratin, polyvinylamine, polyethyleneimine, a copolymer of vinylamine and vinylformamide, a copolymer of acrylamide and 3-methacryloylaminopropyl trimethylammonium, a 3-acrylamidopropyl trimethylammonium polymer or its copolymer, a diallyldimethylammonium-chloride polymer and its copolymer, a polysaccharide with saccharide unit functionalized with hydroxypropyl trimmonium, ethyltrimonium chloride methacrylate and hydrolyzed wheat protein copolymer, alkyl-monium hydroxypropyl hydrolyzed protein, and combinations thereof.

## Patentansprüche

1. Verfahren zur Herstellung biologisch abbaubarer Mikrokapseln, wobei die Mikrokapseln einen Kern und eine Hülle aufweisen, der Kern ein Kernmaterial, das unter Duftstoffen ausgewählt ist, umfasst, die Hülle Erbsenprotein und Alginat umfasst;
wobei das Verfahren keine chemisch kovalente Vernetzung einbezieht, wobei das Verfahren vorzugsweise unter ionischer Vernetzung, Vernetzung mit geladenen Polymeren oder Wasserstoffbrückenbindung, bevorzugter ionischer Vernetzung, ausgewählt ist.

2. Verfahren zur Herstellung von Mikrokapseln nach Anspruch 1, wobei das Verfahren unter ionischer Vernetzung, Vernetzung mit geladenen Polymeren oder Wasserstoffbrückenbindung, vorzugsweise ionischer Vernetzung, ausgewählt ist.

3. Verfahren zur Herstellung von Mikrokapseln nach Anspruch 1 oder Anspruch 2, wobei die Mikrokapseln biologisch abbaubare Duftstoffe einkapseln.

4. Verfahren zur Herstellung von Mikrokapseln nach einem der vorhergehenden Ansprüche, wobei die Mikrokapseln 1 bis 80 Gew.-%, vorzugsweise 2 bis 70 Gew.- % Hüllmaterial umfassen.

5. Verfahren zur Herstellung von Mikrokapseln nach einem der vorhergehenden Ansprüche, wobei die Mikrokapseln ferner ein an der Hülle der Mikrokapsel haftendes Abscheidungshilfsmittel umfassen, wobei das Abscheidungshilfsmittel vorzugsweise aus der Gruppe ausgewählt ist, bestehend aus Trimonium, Methacrylamidopropyltrimethylammonium, Acrylamidopropyltrimethylammonium, Acrylamid, Acrylsäure, Dimethylammonium, Xylose, Galactose, hydroxypropoxylierter Glucose, Chitosan, hydroxyethylierter Glucose, hydroxymethylierter Glucose, Vinylamin, Ethylenimin, funktionalisiertem verzweigtem Polyethylenimin, Vinylformamid, Vinylpyrrolidon, Caprolacton, Catechol, Vinylalkohol, Chitosan, Polyquatemium-4, Polyquatemium-5, Polyquatemium-6, Polyquatemium-7, Polyquatemium-10, Polyquatemium-11, Polyquatemium-16, Polyquatemium-22, Polyquatemium-24, Polyquatemium-28, Polyquatemium-37, Polyquatemium-39, Polyquatemium-44, Polyquatemium-46, Polyquatemium-47, Polyquatemium-53, Polyquatemium-55, Polyquatemium-67, Polyquatemium-68, Polyquatemium-69, Polyquatemium-73, Polyquatemium-74, Polyquatemium-77, Polyquatemium-78, Polyquatemium-79, Polyquatemium-80, Polyquatemium-81, Polyquatemium-82, Polyquatemium-86, Polyquatemium-88, Polyquatemium-101, Polyquatemium-79, hydrolysiertem Keratin, Polyvinylamin, Polyethylenimin, einem Copolymer aus Vinylamin und Vinylformamid, einem Copolymer aus Acrylamid und 3-Methacryloylaminopropyltrimethylammonium, einem 3-Acrylamidopropyltrimethylammoniumpolymer oder dessen Copolymer, einem Diallyldimethylammoniumchloridpolymer und dessen Copolymer, einem Polysaccharid mit einer mit Hydroxypropyltrimonium funktionalisierten Saccharid-Einheit, Ethyltrimoniumchloridmethacrylat und hydrolysiertem Weizenprotein-Copolymer, Alkyl-moniumhydroxypropyl-hydrolysiertem Protein und Kombinationen davon.

## Revendications

1. Procédé de production de microcapsules biodégradables, dans lequel les microcapsules ont un noyau et une enveloppe ; le noyau comprenant une substance de noyau choisi parmi des parfums ; l'enveloppe comprenant une protéine de pois et de l'alginate ;
dans lequel le procédé n'implique pas de réticulation chimiquement covalente, de préférence le procédé est choisi parmi la réticulation ionique, la réticulation de polymères chargés ou la liaison hydrogène, de préférence la réticulation ionique.

2. Procédé de production de microcapsules selon la revendication 1, dans lequel le procédé est choisi parmi la réticulation ionique, la réticulation de polymères chargés ou la liaison hydrogène, de préférence la réticulation ionique.

3. Procédé de production de microcapsules selon la revendication 1 ou 2, dans lequel les microcapsules encapsulent des parfums biodégradables.

4. Procédé de production de microcapsules selon l'une quelconque des revendications précédentes, dans lequel les microcapsules comprennent 1 à 80 % en poids, de préférence 2 à 70 % en poids, de substance d'enveloppe.

5. Procédé de production de microcapsules selon l'une quelconque des revendications précédentes, dans lequel les microcapsules comprennent en outre un adjuvant de dépôt fixé à l'enveloppe de ladite microcapsule, de préférence l'adjuvant de dépôt est choisi dans le groupe constitué par trimonium, méthacrylamidopropyl triméthylammonium, acrylamidopropyltriméthylammonium, acrylamide, acide acrylique, diméthylammonium, xylose, galactose, glucose hydroxypropylé, chitosane,
glucose hydroxyéthylé, glucose hydroxyméthylé, vinylamine, éthylèneimine, polyéthylèneimine ramifiée fonctionnalisée, vinylformamide, vinylpyrollidone, caprolactone, catéchol, alcool vinylique, polyquaternium-4, polyquaternium-5, polyquaternium-6, polyquaternium-7, polyquaternium-10, polyquaternium-11, polyquaternium-16, polyquaternium-22, polyquaternium-24, polyquaternium-28, polyquaternium-37, polyquaternium-39, polyquaternium-44, polyquaternium-46, polyquaternium-47, polyquaternium-53, polyquaternium-55, polyquaternium-67, polyquaternium-68, polyquaternium-69, polyquaternium-73, polyquaternium-74, polyquaternium-77, polyquaternium-78, polyquaternium-79, polyquaternium-80, polyquaternium-81, polyquaternium-82, polyquaternium-86, polyquaternium-88, polyquaternium-101, polyquaternium-79, kératine hydrolysée, polyvinylamine, polyéthylèneimine, un copolymère de vinylamine et vinylformamide, un copolymère d'acrylamide et de 3-méthacryloylaminopropyl triméthylammonium, un polymère de 3-acrylamidopropyl triméthylammonium ou son copolymère, un polymère de chlorure de diallyldiméthylammonium et son copolymère, un polysaccharide avec une unité saccharide fonctionnalisée avec de l'hydroxypropyl trimonium, le chlorure d'éthyl trimonium méthacrylate et un copolymère de protéines de blé hydrolysées, une protéine hydrolysée d'alkyl-monium hydroxypropyl, et leurs combinaisons.
